(19) Europäisches Patentamt
European Patent Office
Office européen des brevets

(11) Publication number: 0 249 140
A2

# (12) EUROPEAN PATENT APPLICATION

(21) Application number: 87108038.8

(22) Date of filing: 03.06.87

(51) Int. Cl.[3]: C 07 C 69/96
C 07 C 68/02

(30) Priority: 09.06.86 US 871984

(43) Date of publication of application:
16.12.87 Bulletin 87/51

(84) Designated Contracting States:
DE FR GB IT NL

(71) Applicant: GENERAL ELECTRIC COMPANY
1 River Road
Schenectady New York 12305(US)

(72) Inventor: Silva, James Manio
8 Barkwood Lane
Clifton Park New York 12065(US)

(72) Inventor: Shannon, Thomas Gerard
1187 Hillside Avenue Apt. 3/B30
Schenectady New York 12309(US)

(72) Inventor: Brunelle, Daniel Joseph
11 Lee Road
Scotia New York 12302(US)

(74) Representative: Catherine, Alain et al,
General Electric - Deutschland Munich Patent Operation
Frauenstrasse 32
D-8000 München 5(DE)

## (54) Method for preparing aromatic bischloroformate compositions.

(57) Aromatic bischloroformate compositions are prepared by concurrently feeding to a back-mixed reactor a substantially insoluble dihydroxyaromatic compound, phosgene, a substantially inert organic liquid and an aqueous alkali or alkaline earth metal base solution. The pH is maintained in the range of about 2-8 and the mixture is agitated so as to ensure intimate admixture of the reagents. The reaction may be conducted batchwise or continuously; if it is conducted continuously, it is sometimes advantageous to further treat the liquid portion of the reaction mixture with base and, optionally, phosgene in a second reactor.

EP 0 249 140 A2

# METHOD FOR PREPARING AROMATIC BISCHLOROFORMATE COMPOSITIONS

This invention relates to the preparation of aromatic bischloroformate compositions.

Aromatic bischloroformates are a known class of organic intermediates useful, for example, for the production of polycarbonates. They may also be used in the preparation of cyclic polycarbonate oligomers which may be converted to very high molecular weight polycarbonates, as disclosed in copending, commonly owned application Serial No. 704,122, filed February 22, 1985, and European Patent Application 162,379, the disclosures of which are incorporated by reference herein, and Serial No. [RD-16741].

In copending, commonly owned application Serial 790,909, filed October 24, 1985, there are disclosed certain bischloroformate compositions and a method for their preparation. According to this method, phosgene is passed into a mixture of a substantially inert organic liquid and a dihydroxy (typically dihydroxyaromatic) compound and there is simultaneously introduced an aqueous alkali metal or alkaline earth metal base solution. The pH of the aqueous phase is maintained in the range of 0.5-8, a relatively low pH value as compared with prior art methods which generally employ a pH of 9 or higher. By this method, bischloroformate compositions are produced which contain relatively large proportions of monomeric bischloroformate as compared with oligomeric carbonate bischloroformates. In particular, said compositions contain relatively small amounts of tetramer and higher carbonate bischloroformates.

Such bischloroformate compositions are particularly useful for the preparation of cyclic polycarbonate oligomers. However, it is of interest to increase still

further the proportion of monomer bischloroformate, since it is often found that conversion to cyclics increases in direct relation to said proportion and decreases with an increase in oligomer bischloroformates, especially those having a degree of polymerization greater than 3. Moreover, said compositions often contain high levels of monochloroformate, which are somewhat less readily converted to cyclics.

A principal object of the present invention, therefore, is to provide an improved method for producing compositions comprising bischloroformates of dihydroxyaromatic compounds.

A further object is to provide a method for preparing bischloroformate compositions containing relatively small proportions of higher oligomeric carbonate bischloroformates and of monochloroformates.

A still further object is to provide a method which is adaptable to batch or continuous operation.

Other objects will in part be obvious and will in part appear hereinafter.

Accordingly, the present invention is a method for preparing an aromatic bischloroformate composition which comprises concurrently feeding to a back-mixed reactor

(A) at least one material selected from the group consisting of dihydroxyaromatic compounds which are substantially insoluble in aqueous systems at said temperature and a pH of about 2-5 and aqueous solutions of bis-alkali metal salts thereof,

(B) phosgene,

(C) at least one substantially inert organic liquid in which the solubility of said dihydroxyaromatic compound is up to about 0.25 M at said temperature, and

(D) an aqueous alkali or alkaline earth metal base solution,

the molar ratio of total phosgene to total reagent A dissolved in said mixture being at least about 1.3:1 and the proportion of reagent D being such as to maintain the pH of the aqueous phase of said mixture in the range of about 2-8; and agitating said mixture so as to ensure intimate admixture of said reagents.

In general, the bischloroformate compositions prepared by the method of this invention consist essentially of compounds having the formula

$$(I) \qquad Cl\left(\overset{O}{\overset{\|}{C}}-O-R-O\right)_n\overset{O}{\overset{\|}{C}}-Cl \quad ,$$

wherein R is a divalent aromatic radical and n is at least 1. It is often desirable to maximize the proportion of monomer bischloroformate (n = 1) therein at the expense of dimer and higher oligomer bischloroformates, monochloroformates, unreacted dihydroxyaromatic compounds and other by-products.

These bischloroformate compositions are prepared from dihydroxyaromatic compounds having the formula HO-R-OH. The R values may be aromatic hydrocarbon or substituted aromatic hydrocarbon radicals, with illustrative substituents being alkyl, cycloalkyl, alkenyl (e.g., crosslinkable-graftable moieties such as vinyl and allyl), halo (especially fluoro, chloro and/or bromo), nitro and alkoxy.

The preferred R values have the formula

$$(II) \qquad -A^1-Y-A^2- \quad ,$$

wherein each of $A^1$ and $A^2$ is a single-ring divalent aromatic radical and Y is a bridging radical in which one or two atoms separate $A^1$ from $A^2$. The free valence bonds in formula II are usually in the meta or para positions of $A^1$ and $A^2$ in relation to Y.

In formula II, the $A^1$ and $A^2$ values may be unsubstituted phenylene or substituted derivatives thereof wherein the substituents are as defined for R. Unsubstituted phenylene radicals are preferred. Both $A^1$ and $A^2$ are preferably p-phenylene, although both may be o- or m-phenylene or one o- or m-phenylene and the other p-phenylene.

The bridging radical, Y, is one in which one or two atoms, preferably one, separate $A^1$ from $A^2$. It is most often a hydrocarbon radical and particularly a saturated $C_{1-12}$ aliphatic or alicyclic radical such as methylene, cyclohexylmethylene, 2-[2.2.1]bicycloheptylmethylene, ethylene, ethylidene, 2,2-propylidene, 1,1-(2,2-dimethylpropylidene), cyclohexylidene, cyclopentadecylidene, cyclododecylidene or 2,2-adamantylidene, especially an alkylidene radical. Aryl-substituted radicals are included, as are unsaturated radicals and radicals containing atoms other than carbon and hydrogen; e.g., oxy groups. Substituents such as those previously enumerated may be present on the aliphatic, alicyclic and aromatic portions of the Y group. For reasons of availability and particular suitability for the purposes of this invention, the preferred radical of formula II is the 2,2-bis(4-phenylene)propane radical, which is derived from bisphenol A and in which Y is 2,2-propylidene and $A^1$ and $A^2$ are each p-phenylene.

For the most part, the suitable compounds include biphenols and especially bisphenols. Frequent reference will be made to bisphenols hereinafter, but it should be

understood that other compounds equivalent thereto may be employed as appropriate.

The following dihydroxyaromatic compounds are illustrative:

Resorcinol
4-Bromoresorcinol
Hydroquinone
4,4'-Dihydroxybiphenyl
1,6-Dihydroxynaphthalene
2,6-Dihydroxynaphthalene
Bis(4-hydroxyphenyl)methane
Bis(4-hydroxyphenyl)diphenylmethane
Bis(4-hydroxyphenyl)-1-naphthylmethane
1,1-Bis(4-hydroxyphenyl)ethane
1,2-Bis(4-hydroxyphenyl)ethane
1,1-Bis(4-hydroxyphenyl)-1-phenylethane
2,2-Bis(4-hydroxyphenyl)propane ("bisphenol A")
2-(4-Hydroxyphenyl)-2-)3-hydroxyphenyl) propane
2,2-Bis(4-hydroxyphenyl)butane
1,1-Bis(4-hydroxyphenyl)isobutane
1,1-Bis(4-hydroxyphenyl)cyclohexane
1,1-Bis(4-hydroxyphenyl)cyclododecane
Trans-2,3-bis(4-hydroxyphenyl)-2-butene
2,2-Bis(4-hydroxyphenyl)adamantane
α,α'-Bis(4-hydroxyphenyl)toluene
Bis(4-hydroxyphenyl)acetonitrile
2,2-Bis(3-methyl-4-hydroxyphenyl)propane
2,2-Bis(3-ethyl-4-hydroxyphenyl)propane
2,2-Bis(3-n-propyl-4-hydroxyphenyl)propane
2,2-Bis(3-isopropyl-4-hydroxyphenyl)propane
2,2-Bis(3-sec-butyl-4-hydroxyphenyl)propane

2,2-Bis(3-t-butyl-4-hydroxyphenyl)propane
2,2-Bis(3-cyclohexyl-4-hydroxyphenyl)propane
2,2-Bis(3-allyl-4-hydroxyphenyl)propane
2,2-Bis(3-methoxy-4-hydroxyphenyl)propane
2,2-Bis(3,5-dimethyl-4-hydroxyphenyl)propane
2,2-Bis(2,3,5,6-tetramethyl-4-hydroxyphenyl)-propane
2,2-Bis(3-5-dichloro-4-hydroxyphenyl)propane
2,2-Bis(3,5-dibromo-4-hydroxyphenyl)propane
2,2-Bis(2,6-dibromo-3,5-dimethyl-4-hydroxyphenyl)propane
α,α-Bis(4-hydroxyphenyl)toluene
α,α,α',α'-Tetramethyl-α,α'-bis(4-hydroxyphenyl)-p-xylene
2,2-Bis(4-hydroxyphenyl)hexafluoropropane
1,1-Dichloro-2,2-bis(4-hydroxyphenyl)ethylene
1,1-Dibromo-2,2-bis(4-hydroxyphenyl)ethylene
1,1-Dichloro-2,2-bis(5-phenoxy-4-hydroxyphenyl)ethylene
4,4'-Dihydroxybenzophenone
3,3-Bis(4-hydroxyphenyl)-2-butanone
1,6-Bis(4-hydroxyphenyl)-1,6-hexanedione
Bis(4-hydroxyphenyl) ether
Bis(4-hydroxyphenyl) sulfide
Bis(4-hydroxyphenyl) sulfoxide
Bis(4-hydroxyphenyl) sulfone
Bis(3,5-dimethyl-4-hydroxyphenyl) sulfone
9,9-Bis(4-hydroxyphenyl)fluorene
2,7-Dihydroxypyrene
6,6'-Dihydroxy-3,3,3',3'-tetramethylspiro-(bis)indane ("spirobiindane bisphenol")
3,3-Bis(4-hydroxyphenyl)phthalide
2,6-Dihydroxydibenzo-p-dioxin

2,6-Dihydroxythianthrene
2,7-Dihydroxyphenoxathiin
2,7-Dihydroxy-9,10-dimethylphenazine
3,6-Dihydroxydibenzofuran
3,6-Dihydroxydibenzothiophene
2,7-Dihydroxycarbazole.

The preferred dihydroxyaromatic compounds are those which are substantially insoluble in aqueous systems at temperatures within the range of 20-40°C and pH values in the range of about 2-5. Thus, dihydroxyaromatic compounds of relatively low molecular weight and high solubility in water, such as resorcinol and hydroquinone, are generally less preferred. Bisphenol A is often especially preferred for reasons of availability and particular suitability for the purposes of the invention.

According to the invention, reagent A is at least one material selected from the group consisting of the dihydroxyaromatic compounds described above and aqueous solutions of bis-alkali metal salts thereof. The bis-alkali metal salts may be lithium, sodium, potassium, rubidium or cesium salts, with sodium and potassium and especially sodium generally being preferred because of relative availability and low cost. Upon contact with the organic liquid phase under the prevailing conditions of relatively low pH, the salt is converted to the free bisphenol, most of which precipitates in finely divided condition and a small amount of which dissolves in the organic phase and is available for conversion to bischloroformate. In general, however, it is preferred to employ the free dihydroxyaromatic compounds rather than their salts, since the use of the salts requires a relatively large amount of water to ensure their complete dissolution and may thus cause hydrolysis of phosgene.

Reagent B is phosgene, and reagent C is at least one substantially inert organic liquid. The solubility of the bisphenol in reagent C is also an important factor. Such solubility should be up to about 0.25 M at temperatures in the range of about 20-40°C, and preferably up to about 0.1 M. Said liquid should generally also be substantially insoluble in water. Illustrative liquids are aliphatic hydrocarbons such as hexane and n-heptane; chlorinated aliphatic hydrocarbons such as methylene chloride, chloroform, carbon tetrachloride, dichloroethane, trichloroethane, tetrachloroethane, dichloropropane and 1,2-dichloroethylene; aromatic hydrocarbons such as benzene, toluene and xylene; substituted aromatic hydrocarbons such as chlorobenzene, o-dichlorobenzene, the chlorotoluenes, nitrobenzene and acetophenone; and carbon disulfide. The chlorinated aliphatic hydrocarbons, especially methylene chloride, are preferred.

Reagent D, the aqueous alkali or alkaline earth metal base solution, is most often a hydroxide such as sodium hydroxide, potassium hydroxide or calcium hydroxide. Sodium and potassium hydroxides, and especially sodium hydroxide, are preferred because of their relative availability and low cost.

According to the present invention, the bischloroformate-forming reaction is conducted, at least in part, in a back-mixed reactor. Certain embodiments of the invention also involve a second reactor. The unmodified term "reactor" when used herein will refer to the first or only back-mixed reactor.

Back-mixed reactors include tank and certain types of loop reactors. Tank reactors are particularly preferred; these include continuous-flow stirred tank reactors

(hereinafter "CSTR's"), which are particularly useful when the reactor is conducted continuously as described hereinafter.

The above-described reagents are concurrently fed to the reactor. Reagent A may be introduced as free bisphenol, as a solution of bisphenol in reagent D or, as previously noted, aqueous bisphenol salt.

In a particularly preferred embodiment of the invention, reagent A is added as free bisphenol, either in solid form or as a slurry in reagent C. The latter is frequently advantageous since slurries are generally easier to handle than solids, and reagent C then provides a liquid seal for the reactor.

The rate of addition of phosgene is adjusted so that over the entire course of the reaction, at least about 1.3 moles of total phosgene is added per mole of total reagent A dissolved in the reaction mixture (i.e., excluding solid bisphenol). Assuming complete reaction, the principal products will then be monomer and dimer bischloroformates (mole ratios 2:1 and 1.5:1, respectively). Since a high proportion of monomer bischloroformate is generally desired, a molar ratio of at least about 2:1 is generally preferred. There is no critical upper limit for this mole ratio, but it is seldom advantageous to employ more than 2.5 moles of phosgene per mole of dissolved reagent A, and 2.2 moles or less is usually sufficient.

It is frequently preferred for reagent A to be fed to the reactor in a form that provides a minor proportion of undissolved (normally solid) bisphenol therein; that is, bisphenol in excess of the amount soluble in water and in reagent C. When reagent A is a bisphenol salt solution, solid bisphenol is provided by neutralization of the salt at the low ambient pH; when it is free bisphenol, it is fed in

amounts greater than the solubility thereof. In this way, the dissolution of a relatively small and constant proportion of bisphenol in the mixture for reaction with phosgene is assured. Since the reaction is relatively rapid, it is usual and preferred for the proportion of dissolved reagent A in the mixture at any time to be less than the saturation level thereof.

This is in contrast to the method described in the aforementioned application Serial No. 790,909, in which all of the bisphenol is initially present in the reaction vessel for contact with the phosgene. Under those conditions, the initial high proportion of bisphenol may promote the formation of oligomer carbonate bischloroformates rather than monomer bischloroformates. By the present method, on the other hand, it is a simple matter to maintain the desired high molar proportion of phosgene at all stages of the reaction.

At the beginning of the run, some water and reagent C should be present in the reactor. It is within the scope of the invention to begin introduction of reagents A and B simultaneously, or to feed reagents B and C initially for a short period and then begin to introduce reagent A. Phosgene addition may be at a constant or variable rate, provided reactant proportions are maintained within the previously stated ranges.

The pH of the aqueous phase of the reaction mixture is maintained within the range of about 2-8, preferably about 2-5, by addition of suitable amounts of reagent D. Said pH level may be kept substantially constant or may be programmed within the prescribed range during the course of the reaction.

The proportion of reagent D required for this purpose can readily be determined by routine experimentation; it is usually about 2-3 equivalents per mole of bisphenol. The concentration of reagent D is not critical and may be, for example, about 1-19 M.

The reaction pressure is usually atmospheric, although it is possible to operate at other (particularly increased) pressures. At higher or lower pressures, higher or lower temperatures may be employed. The temperature under atmospheric pressure conditions is usually maintained within the range of 10-40°C, most often 20-40°C. It is frequently advantageous to maintain a maximum temperature of 30°C. However, under certain conditions a higher temperature may have advantage, particularly when reagent C is methylene chloride whereupon the reaction may be conducted at reflux (about 39°C).

The reaction mixture prepared as described above is efficiently agitated to ensure intimate admixture. Agitation may be conveniently achieved by use of conventional equipment, typically a turbine stirrer or the like when a tank reactor is used. It is important that the entire reaction mixture be effectively agitated to maximize bisphenol conversion.

The degree of completion of conversion of bisphenol in the reactor will depend on various factors, including those discussed hereinabove and others such as reaction time or residence time and reactor design. Suitable reaction or residence times are generally on the order of 5 minutes to 1-1/2 hours; when the process is run continuously, a residence time of about 5-30 minutes is usually adequate.

If solid or otherwise unreacted bisphenol remains in the mixture after this reaction time or residence time, the reaction has obviously not reached completion. It may then be advantageous to continue the reaction in the same

equipment and under the same conditions or to separate solid bisphenol, which may be recycled, and subject the liquid portion of the mixture to further treatment. Solids separation may be achieved by conventional means such as filtration, screening or centrifugation.

In situations where it is desirable to maximize conversion of bisphenol to bischloroformate as opposed to monochloroformate, or to increase the conversion of dissolved bisphenol, any solids in the reaction mixture may be removed and the liquid phase may be further treated with reagent D as necessary to maintain a pH within the previously described range for an additional period, typically up to about 15 minutes. This permits post-phosgenation of dissolved but unreacted bisphenol and monochloroformates, which may be facilitated if desired by the addition of a further amount of phosgene.

Particularly in continuous operations, this further treatment of the liquid reaction mixture is often conveniently conducted in a second reactor. Typically, solid bisphenol is separated by passing the effluent from the first reactor through a fine mesh screen. The second reactor may be either a back-mixed or plug flow reactor, into which the liquid from the first reactor passes and may be supplemented by additional reagent B and/or reagent D as necessary or desired.

The distributions of the molecular species in the bischloroformate compositions prepared by the method of this invention may be determined by reversed phase high pressure liquid-liquid chromatography. The composition is first reacted with an equimolar mixture of phenol and triethylamine to produce the corresponding phenyl esters, which are

resistant to hydrolysis under chromatography conditions. The phenyl esters are dissolved in a mixture of tetrahydrofuran and water and chromatographed using a relatively non-polar packing, whereupon lower molecular weight constituents are eluted first. For each molecular species, two values are determined and used for identification: the retention time (in minutes) and the area under the ultraviolet absorption peak at 254 nm., which is uniquely identifiable for compounds of this type.

The standards used for assignment of retention time and 254 nm. absorption are separately prepared linear compounds including bisphenol A mono- and diphenyl carbonate and the diphenyl carbonate of bisphenol A dimer. Higher oligomers are detected by analogy.

The invention is illustrated by the following examples. Examples 1-4 illustrate batch reactions, and Examples 5-7 various aspects of continuous reactions.

Examples 1-3

A 500-ml. Morton flask was fitted with a paddle-type impeller, pH probe, phosgene addition dip tube, thermometer, condenser and cooling bath. The flask was charged with 150 ml. of methylene chloride, 20 ml. of water, a measured amount of solid bisphenol A and a small amount of benzophenone as an internal standard. The temperature of the mixture was stabilized at 25°C and phosgene was added, with impeller rotation at 300-350 rpm., at a constant rate to a total of 33 grams (0.33 mole). Additional portions of bisphenol A, in the amount originally introduced, were also added incrementally at measured intervals, to a total of 34.2 grams (0.15 mole). The pH of the aqueous phase was

maintained in the range 2-5 by metered addition of 5 M aqueous sodium hydroxide.

After addition of the final increment of bisphenol A, phosgene addition was continued for a period equal to the period between bisphenol A additions, with pH regulation as described above. In Example 2, the mixture was stirred for an additional 10 minutes after completion of phosgene addition, with pH control. The product was analyzed for total monomeric product (monochloroformate and bischloroformate), for total monomeric and oligomeric monochloroformates and for residual bisphenol A and phosgene.

The relevant parameters and product data are given in Table I. The corresponding controls were run by similar procedures except that the entire portion of bisphenol A was introduced into the reaction vessel before phosgene addition was begun.

TABLE I

| | Ex. 1 | Control 1 | Ex. 2 | Control 2 | Ex. 3 | Control 3 |
|---|---|---|---|---|---|---|
| Bisphenol addition period, min. | 15 | 15 | 30 | 30 | 70 | 70 |
| Interval between bisphenol increments, min. | 1 | -- | 2 | -- | 2 | -- |
| Phosgene addition rate: g./min. | 2.18 | 2.18 | 1.09 | 1.09 | 0.47 | 0.47 |
| mmol./min. | 22 | 22 | 11 | 11 | 4.71 | 4.71 |
| % monomer | 59 | 55 | 53.5 | 40.9 | 49.6 | 39.9 |
| % monochloroformate | 25.9 | 20.2 | 22.4 | 15.1 | 19.3 | 16.3 |
| Unreacted bisphenol, mmol./l. | 41 | 12 | 0 | 0 | 12 | 0 |
| Unreacted phosgene, mmol./l. | 390 | 320 | 150 | 50 | 43.5 | 125 |

The results of these examples show the substantial increase in proportion of monomeric product obtained according to the method of this invention. This is accompanied by an increase in proportion of monochloroformate products; however, these may be converted to the corresponding bischloroformates by one of the methods described hereinabove. Example 2 shows the advantage of continued pH control with respect to consumption of unreacted bisphenol, and all three examples show the presence at the end of the reaction of sufficient phosgene to convert any unreacted bisphenol to product if desired.

Example 4

This example shows the necessity of effective agitation. The procedure of Example 2 was repeated, replacing the impeller with a smaller impeller rotated at 300 rpm. and continuing phosgene addition for 10 minutes after the completion of the 30-minute reaction period. It was noted that the top surface of the liquid in the reactor was nearly quiescent. A total of 3.29 grams of unreacted bisphenol A was recovered, as compared with total reaction of the bisphenol A in Example 2.

Example 5

The reactor and equipment were similar to those used in Examples 1-3, except for the presence of an overflow port in the flask at a level to provide a working reactor volume of 170 ml. The reactor was initially charged with 15 grams (66 mmol.) of solid bisphenol A, 130 ml. of methylene chloride and 20 ml. of water. Phosgene was added at 0.87 gram (8.79 mmol.) per minute at a temperature below 28°C and

for the most part in the range 22-25°C, and the pH was maintained in the range 2-5 by addition of 4.75 M aqueous sodium hydroxide solution at the rate of about 2 ml. per minute. Efficient agitation of the entire reaction mixture was provided by operating the stirrer (a single paddle impeller) at 275 rpm.

The reaction was conducted batchwise for 10 minutes, after which methylene chloride was fed continuously into the reactor at 16 ml. per minute and solid bisphenol A was added in 15-gram increments at 5-10 minute intervals to ensure its presence at all times in the reactor. These conditions provided a residence time of 10 minutes.

The overflow from the reactor, comprising solid bisphenol A and two liquid phases, was analyzed at intervals for monomer, dimer, trimer and tetramer bischloroformate and monomer and dimer monochloroformate. The results are given in Table II. All composition figures are in percent by weight of total product.

TABLE II

| | Reaction time, min. | | | |
|---|---|---|---|---|
| | 10 | 20 | 30 | 45 |
| Monomer bischloroformate | 22.7 | 40.7 | 55.3 | 60.9 |
| Monomer monochloroformate | 41.5 | 26.6 | 23.3 | 22.4 |
| Dimer bischloroformate | 14.4 | 15.8 | 12.1 | 9.4 |
| Dimer monochloroformate | 14.9 | 10.4 | 6.8 | 6.0 |
| Trimer bischloroformate | 4.8 | 5.1 | 2.5 | 1.3 |
| Tetramer bischloroformate | 1.7 | 1.4 | ---- | ---- |

Example 6

The procedure was identical to that of Example 5, except that the phosgene flow rate was doubled to 1.74 grams (17.58 mmol.) per minute and the temperature was allowed to

rise to 35°C during the batchwise portion of the reaction. The results are given in Table III.

TABLE III

| | Reaction time, min. | | | | |
|---|---|---|---|---|---|
| | 14 | 30 | 40 | 50 | 60 |
| Monomer bischloroformate | 75.4 | 75.3 | 67.8 | 75.5 | 65.6 |
| Monomer monochloroformate | 6.2 | 13.5 | 14.4 | 13.6 | 16.8 |
| Dimer bischloroformate | 10.3 | 8.9 | 9.3 | 9.3 | 8.5 |
| Dimer monochloroformate | 5.0 | * | 6.3 | * | 7.0 |
| Trimer bischloroformate | 2.4 | 1.7 | 1.8 | 1.6 | 1.6 |
| Tetramer bischloroformate | 0.7 | 0.6 | 0.4 | ---- | 0.5 |

*Undetermined minor amount; peak appears as shoulder on monomer bischloroformate peak.

Example 7

This example models the use of a second reactor for further reaction of the liquid reaction mixture from the first reactor.

A reaction vessel similar to that of Examples 1-3 was charged with 15 grams of bisphenol A, 130 ml. of methylene chloride and 20 ml. of water and phosgene was added at 15-25°C, with stirring at 275 rpm., until all solid bisphenol A had been consumed, with maintenance of the pH in the range 2-5 by metered addition of 9.5 M aqueous sodium hydroxide solution. The total time required was about 12-14 minutes. Phosgene passage and pH control were then continued and the mixture was periodically sampled and analyzed. The results are given in Table IV.

TABLE IV

| | Reaction time, min. | | | |
|---|---|---|---|---|
| | 10* | 15 | 20 | 25 |
| Monomer bischloroformate | 36.2 | 44.4 | 46.1 | 46.1 |
| Monomer monochloroformate | 8.0 | 3.8 | 2.1 | 2.0 |
| Dimer bischloroformate | 22.5 | 22.7 | 24.1 | 24.1 |
| Dimer monochloroformate | 6.5 | 4.0 | ---- | ---- |
| Trimer bischloroformate | 13.6 | 13.7 | 14.3 | 14.0 |
| Higher bischloroformates | 13.2 | 11.4 | 13.4 | 13.8 |

*Simulates liquid phase of effluent from first reactor.

It is apparent from the results in Table IV that a second reactor is effective to convert monochloroformate to bischloroformate.

What is claimed is:

1. A method for preparing an aromatic bischloroformate composition which comprises concurrently feeding to a back-mixed reactor

(A) at least one material selected from the group consisting of dihydroxyaromatic compounds which are substantially insoluble in aqueous systems at said temperature and a pH of about 2-5 and aqueous solutions of bis-alkali metal salts thereof,

(B) phosgene,

(C) at least one substantially inert organic liquid in which the solubility of said dihydroxyaromatic compound is up to about 0.25 M at said temperature, and

(D) an aqueous alkali or alkaline earth metal base solution,

the molar ratio of total phosgene to total reagent A dissolved in said mixture being at least about 1.3:1 and the proportion of reagent D being such as to maintain the pH of the aqueous phase of said mixture in the range of about 2-8; and agitating said mixture so as to ensure intimate admixture of said reagents.

2. A method according to claim 1 wherein some water and reagent C are initially present in the reactor and the pH is maintained in the range of about 2-5.

3. A method according to claim 2 wherein the pressure is atmospheric and the temperature is maintained in the range of about 10-40°C.

4. A method according to claim 3 wherein reagent A is said dihydroxyaromatic compound.

5. A method according to claim 4 wherein reagent A is added in solid form or as a slurry in reagent C.

6. A method according to claim 5 wherein the back-mixed reactor is a tank reactor.

7. A method according to claim 6 wherein the dihydroxyaromatic compound has the formula $HO-A^1-Y-A^2-OH$, wherein each of $A^1$ and $A^2$ is a single-ring divalent aromatic radical and Y is a bridging radical in which 1 or 2 atoms separate $A^1$ from $A^2$.

8. A method according to claim 7 wherein reagent C is methylene chloride and reagent D is an aqueous sodium hydroxide solution.

9. A method according to claim 8 wherein reagent A is bisphenol A.

10. A method according to claim 6 wherein the reaction is conducted batchwise.

11. A method according to claim 10 wherein the dihydroxyaromatic compound has the formula $HO-A^1-Y-A^2-OH$, wherein each of $A^1$ and $A^2$ is a single-ring divalent aromatic radical and Y is a bridging radical in which 1 or 2 atoms separate $A^1$ from $A^2$.

12. A method according to claim 11 wherein reagent C is methylene chloride and reagent D is an aqueous sodium hydroxide solution.

13. A method according to claim 12 wherein reagent A is bisphenol A and is added as a slurry in reagent C.

14. A method according to claim 13 wherein the reaction is continued until no undissolved bisphenol A remains, after which the reaction mixture is further treated with reagent D as necessary to maintain a pH within the range of 2-5 for an additional period.

15. A method according to claim 6 wherein the back-mixed reactor is a continuous stirred tank reactor and the reaction is conducted continuously.

16. A method according to claim 15 wherein solid reagent A is separated from the effluent from the continuous stirred tank reactor and the liquid reaction mixture thus

obtained is further treated with reagent D in a second reactor.

17. A method according to claim 16 wherein the mixture is also treated with reagent B in said second reactor.

18. A method according to claim 17 wherein the dihydroxyaromatic compound has the formula $HO-A^1-Y-A^2-OH$, wherein each of $A^1$ and $A^2$ is a single-ring divalent aromatic radical and Y is a bridging radical in which 1 or 2 atoms separate $A^1$ from $A^2$.

19. A method according to claim 18 wherein reagent C is methylene chloride and reagent D is an aqueous sodium hydroxide solution.

20. A method according to claim 19 wherein reagent A is bisphenol A and is added as a slurry in reagent C.

WHP/tg